# EUROPEAN PATENT SPECIFICATION

(11) **EP 3 041 459 B1**
(45) Date of publication and mention of the grant of the patent: **03.04.2019**
(21) Application number: 14786305.4
(22) Date of filing: 03.09.2014
(51) Int. Cl.: A61K 8/36, A61K 8/37, A61K 31/202, A61K 31/232, A61K 9/00, A61K 47/10, A61K 47/12, A61K 47/26, A61K 47/44, A61P 17/16, A61P 35/00

(54) **COMPOSITIONS USEFUL IN THE PREVENTION OR TREATMENT OF SKIN CANCER**
ZUSAMMENSETZUNGEN ZUR PRÄVENTION ODER BEHANDLUNG VON HAUTKREBS
COMPOSITIONS UTILES DANS LE TRAITEMENT PROPHYLACTIQUE OU THÉRAPEUTIQUE DU CANCER DE LA PEAU

(30) Priority: 06.09.2013 IT MI20131475
(43) Date of publication of application: 13.07.2016
(73) Proprietor: Giuliani S.p.A., 20129 Milano (IT)
(72) Inventor: GIULIANI, Giammaria, I-20123 Milano (IT); BENEDUSI, Anna, I-20124 Milano (IT); MARZANI, Barbara, I-27020 Carbonara Al Ticino (IT); BARONI, Sergio, I-24030 Villa d'Adda (IT); PICARDO, Mauro, I-00198 Roma (IT)
(74) Representative: Gervasi, Gemma
(86) International application number: PCT/IB2014/064223
(87) International publication number: WO 2015/033276

(56) References cited:
- EP-A1- 2 407 163
- FLORI ENRICA ET AL: "2,4,6-Octatrienoic acid is a novel promoter of melanogenesis and antioxidant defence in normal human melanocytes via PPAR-[gamma] activation.", PIGMENT CELL & MELANOMA RESEARCH AUG 2011, vol. 24, no. 4, August 2011 (2011-08), pages 618-630, XP002720920, ISSN: 1755-148X
- FLORI ENRICA ET AL: "2,4,6-octatrienoic acid promotes melanogenesis in normal human melanocytes", JOURNAL OF INVESTIGATIVE DERMATOLOGY, vol. 130, no. Suppl. 2, September 2010 (2010-09), page S98, XP002720921, & 40TH ANNUAL MEETING OF THE EUROPEAN-SOCIETY-FOR-DERMATOLOGICAL-RESEAR CH (ESDR 2010); HELSINKI, FINLAND; SEPTEMBER 08 -11, 2010 ISSN: 0022-202X
- BRIGANTI STEFANIA ET AL: "2,4,6-octatrienol, a polyunsaturated alcohol, counteracts molecular markers of skin cell senescence", JOURNAL OF INVESTIGATIVE DERMATOLOGY, vol. 130, no. Suppl. 2, September 2010 (2010-09), page S48, XP002720922, & 40TH ANNUAL MEETING OF THE EUROPEAN-SOCIETY-FOR-DERMATOLOGICAL-RESEAR CH (ESDR 2010); HELSINKI, FINLAND; SEPTEMBER 08 -11, 2010 ISSN: 0022-202X

## Description

### Field of the Invention

The object of the present invention is a novel anticancer use of known compounds, and compositions thereof which contain said compounds as active ingredient.

### Background of the Invention

In each cell, the DNA is the repository of the genetic information; its stability and integrity are essential to life. The DNA is subject to environmental insults and any consequent damage, if not repaired, leads to a mutation and, possibly, to the onset of a disease state.

Skin cancer from damage caused by excessive exposure to ultraviolet radiation is an example of the link between environmental damage induced to the DNA and the onset of a disease.

Another example is the DNA damage caused by tobacco smoke, which may thus lead to mutations in the lung cells and subsequent lung cancer.

In addition to the attack of environmental agents, the DNA is also subject to damage caused by metabolism by-products.

Whatever the origin of the harmful attack, it is estimated that a single cell can suffer up to a million DNA changes in a day. In addition to the damage caused by environmental insults or metabolites, the process of DNA replication during cell division is subject to errors itself. The speed at which the DNA polymerase adds not correct nucleotides during the DNA replication is an important factor in determining the rate of spontaneous mutation in an organism.

Although the cell is equipped with enzymatic systems that normally recognize and correct many of these errors, some mutations remain. For a given gene, estimates of the frequency with which the human DNA undergoes errors that are not corrected are roughly between 1 x 10⁻⁴ to 1 x 10⁻⁶ mutations per gamete. A rate of 1 x 10⁶ expresses the possibility of finding a mutation in a specific locus for a million gametes.

The DNA repair processes are present in both prokaryote and eukaryote organisms. Many proteins involved in these processes are highly conserved during evolution because of the importance of this repair function. The cells have developed several mechanisms to detect and correct the various types of damage that can occur to DNA, regardless of the source of damage. Because the DNA is a molecule that plays an active and critical role in cell division, the DNA repair is closely linked to the regulation of the cell cycle.

During the cell cycle, in fact, control mechanisms (checkpoints) ensure that the DNA of a cell is intact before allowing that the DNA replication and the cell division happen. A defect in the checkpoints may lead to an accumulation of damages, which in turn lead to mutations.

Among the harmful environmental factors, for example, it is particular well-known that the ultraviolet radiation (UVA and UVB) is the main etiologic agent in the development of many skin cancers. It causes DNA damage through the formation of pyrimidine cyclobutane dimers (CPD) and pyrimidine(6-4)pyrimidone adducts, or 6-4 photoproducts (6-4 PP). Both compounds are able to modify the DNA structure, thus preventing transcription and replication.

The phosphorylated form of histone γH2AX, the p53 protein and the protein GADD45a (growth arrest and DNA damage response gene), are also cited as known markers of DNA damage induced by UV irradiation.

In addition, 8-oxo-2'-deoxyguanosine (8-oxo-dG or 8-OHdG), an oxidized derivative of deoxyguanosine, is one of the major products of the DNA oxidation and, therefore, another typical marker of damage against it, particularly under exposure to UVA ultraviolet radiation.

The DNA repair is a physiological process that continuously operates in cells to protect the genome from damage and harmful mutations. The damage from CPD dimers and 6-4 PP photoproducts is counteracted through a process known as nucleotide excision repair (NER). In eukaryotes this complex process may repair a damage that involves up to 30 nucleotides.

Defects to some of these genes, if not repaired, can lead for example to genodermatoses, such as xeroderma pigmentosum, as well as other serious pre-cancerous dermatological disorders, *i.e.* characterized by a very high risk of developing skin cancer (about one thousand times greater than normal).

With reference to other technical problems and, therefore, in view of different uses, the same Applicant has in the past studied a class of dienes characterized by a general formula CH₃(-CH=CH)ₙ-R (wherein n=2-7) and, in this respect, is the holder of various publications.

WO2010/052328 suggests for these compounds, investigated with reference to retinol as a positive control, a pharmaceutical or cosmetic use to improve the human epidermis cell repair activity by means of the production of keratin mediated by cytokeratins cK-19. The transcriptional study on the expression of cK-19 showed an increase (up-regulation) of cK-19 in the final differentiation of the human epidermis reconstructed *in vitro*.

WO2010/052329 describes for these compounds, through the definition of an inhibitory action of the enzyme 5α-reductase, a cosmetic use directed to promote the integrity of the skin through the effect of increasing the strength of the collagen and elastin.

WO2011/132177 suggests the use of some of these compounds to counteract the oxidizing action of the free radicals ROS and preserve the physiological conditions of the human epidermis.

WO2012/007572 describes an activity of such compounds in the induction of melanogenesis through a non-receptor route. In particular, experiments performed on primary melanocytes cultures showed the ability to induce the expression and activity of tyrosinase, the main melanogenic enzyme, and to increase the intracellular content of melanin.

The European patent application EP 2 407 163A1 discloses compounds with a skin pigmenting activity and pharmaceutical or cosmetic compositions containing them.

None of these prior art documents relates to the behavior of these diene compounds with reference to the problem of human DNA damage caused by environmental agents, such as those caused by excessive UV radiation may be. In fact, none of the experiments on which the evidence of activity in the uses as described in these prior art is based, contemplate, for example, UV irradiation, and therefore none of them measures the effects of UV radiation as a damage factor to human DNA, and thus as a carcinogen in case of skin cancers that develop through an initial stage of DNA damage caused by UV radiation.

### Summary of the Invention

Experimental studies, reported in the following paragraphs of the present description, have now surprisingly shown that for some of said compounds it is possible to obtain a significant activity of inhibition of the DNA damage produced by UV radiation, thereby envisaging a use for them as anticancer, both as prevention and therapy, in the case of skin cancer induced by this kind of DNA damage.

Object of the invention are therefore compounds of general formula (I):

CH₃-(CH=CH)ₙ-R (I)

wherein n = 3, 5, 7; R is selected from -CO-OR', -CO-O⁽⁻⁾, R' being selected from H, C₁-C₂₂ alkyl or alkenyl, aryl or aralkyl, or sugars;
and pharmaceutically acceptable salts thereof, preferably such as sodium, potassium, lysine salts,
each compound of general formula (I) being used as such or in admixture with one or more of the other ones,
for use in the prevention or treatment of skin cancer resulting from DNA damage produced by UV radiation, wherein the compounds topically administered to the skin produce at least the following combined effects: preservation of cell viability, positivity to annexin V, apoptosis inhibition, inhibition of caspase-3 protein expression, inhibition of CPDs and 8-OHdG.

Among the dermatological disorders to be treated according to such use, for example, xeroderma pigmentosum and photocarcinogenesis are cited as examples.

A secondary use, dependent on the anticancer one, particularly in the case of prevention of DNA damage caused by UV radiation, is envisaged as active ingredient in compositions directed to the photoprotection of the skin from UVA and UVB, specifically to prevent DNA damage.

Therefore, a further object of the present invention is such photoprotective use dependent on the above described use.

The present invention also relates to any composition for the uses described above in both the therapeutic and the prevention field, comprising as active ingredient a compound of general formula (I) as identified above, with any suitable excipient, particularly for topical application on the skin, including the scalp.

### Detailed Description of the Invention

Some preferred compounds corresponding to the formula (I) for the use envisaged in the present invention are:
2,4,6 octatrienoic acid
2,4,6 octatrienoic acid sodium salt, or potassium salt
2,4,6-octatrienoic acid ethyl ester
2,4,6-octatrienoic acid L-lysine salt
2,4,6-octatrien-1-ol acetate

The following examples illustrate the invention.

The formula and the molecular weight for some of the compounds of general formula (I) are shown below. 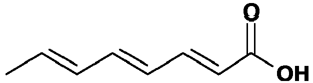
C₈H₁₀O₂M.W.138.17
2,4,6 octatrienoic acid
Sodium salt: C₈H₉O₂Na
Lysin salt: C₈H₉O₂.C₆H₁₅N₂O₂,

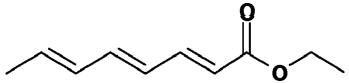

C₁₀H₁₄O₂ M.W. 166.22
2,4,6-octatrienoic acid ethyl ester

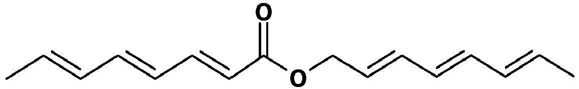

C₁₆H₂₀O₂ M.W. 244.34
2,4,6-octatrienyl ester of 2,4,6-octatrienoic acid

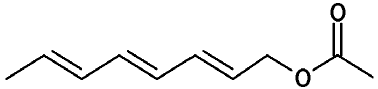

C₁₀H₁₄O₂ M.W. 166.22
2,4,6-octatrien-1-ol acetate

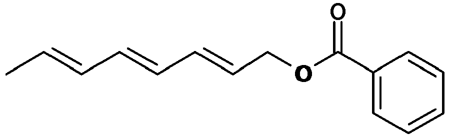

C₁₅H₁₆O₂ M.W.228.29
2,4,6-octatrienyl ester of benzilic acid; 2,4,6-octatrien-1-ol, benzoate In a composition according to the invention, said compound is present as active ingredient in an amount preferably in the range from 10⁻⁶ to 3x10⁻²mol/100g. Examples of compositions particularly suitable for the above-stated use are given below.

The quantities of the components are expressed as percentages by weight:

### Example 1

**ANTICANCER DERMATOLOGICAL PREPARATION**

| Component | Amount (% w/w) |
|---|---|
| 2-Octyldodecanol | 5-20 |
| Cetostearyl alcohol | 5-15 |
| Cetyl esters wax | 1-5 |
| Sorbitan monostearate | 1-5 |
| Polisorbate 60 | 1-5 |
| Benzilic alcohol | 0.1-1 |
| 2,4,6-Octatrienoic acid | 0.1-0.5 |
| Purified water | q.s. to 100 mL |

### Example 2

**DERMATOLOGICAL PREPARATION HIGH PROTECTION AGAINST UV DAMAGE**

| Component | Amount (% w/w) |
|---|---|
| C₂₀-C₂₂ alkyl phosphate | 0.50-5.00 |
| C₂₀-C₂₂ alcohols | 0.50-5.00 |
| Cetearyl glucoside | 0.10-2.00 |
| Tromethamine | 0.10-0.80 |
| Dibutyl adipate | 1.00-7.00 |
| Ethylhexyl methoxycinnamate | 1.00-10.00 |
| Isostearyl isostearate | 2.00-8.00 |
| Ethylhexyl salicylate | 1.00-5.00 |
| Butylene glycol cocoate | 1.00-5.00 |
| Butyl methoxydibenzoylmethane | 1.00-5.00 |
| Diethylamino Hydroxybenzoyl Hexyl Benzoate | 1.00-5.00 |
| Octocrylene | 1.00-5.00 |
| Bis-Ethylhexyloxyphenol Methoxyphenyl Triazine | 1.00-4.00 |
| Diethylhexyl syringylidene malonate | 0.10-1.00 |
| 2,4,6-Octatrienoic acid | 0.05-0.50 |
| Lysine Octatrienoate | 0.05-0.50 |
| Phenoxyethanol | 0.80-1.00 |
| Glycerin | 1.00-5.00 |
| Disodium EDTA | 0.01-0.09 |
| Tocopheryl acetate | 0.05-1.00 |
| Potassium sorbate | 0.005-0.10 |
| Citric acid | q.s. to pH 5.5 |
| Purified water | q.s. to 100 g |

### Example 3

**DERMATOLOGICAL PREPARATION FOR FACE PROTECTION**

| Component | Amount (% w/w) |
|---|---|
| Xanthan gum | 0.10-0.20 |
| Sodium hyaluronate | 0.05-1.00 |
| Inositol | 0.05-1.00 |
| Xylitol | 0.05-1.00 |
| Taurine | 0.05-1.00 |
| Betaine | 0.05-1.00 |
| C₁₄-C₂₂ Alcohols | 0.05-2.00 |
| C₁₂-C₂₀ Alkyl glucoside | 0.05-1.00 |
| Octyldodecanol | 1.00-3.00 |
| Ethylhexyl methoxycinnamate | 0.50-3.00 |
| Octocrylene | 0.10-3.00 |
| Butylene glycol | 0.10-5.00 |
| Glycerin | 1.00-4.00 |
| 2,4,6-Octatrienoic acid | 0.05-0.50 |
| Phenoxyethanol | 0.80-1.00 |
| Sodium hydroxide | 0.001-0.20 |
| Citric acid | 0.001-0.30 |
| Hydroxyethyl acrylate/Sodium acryloyldimethyl | 0.01-2.00 |

| Taurate copolymer | |
|---|---|
| Polyisobutene | 0.01-1.50 |
| PEG-7 Trimethylolpropane Coconut Ether | 0.01-1.00 |
| Purified water | q.s. to 100 |

### Example 4

**PROTECTIVE AND ANTICANCER DERMATOLOGICAL PREPARATION FOR THE SCALP**

| Component | Amount (% w/w) |
|---|---|
| Ethanol | 10.00-30.00 |
| Pentylene glycol | 0.025-0.20 |
| Potassium Octatrienoate | 0.05-0.50 |
| PEG-6 Caprylic/Capric Glycerides | 0.10-1.00 |
| Purified water | q.s. to 100.00 |

### Example 5

**DERMATOLOGICAL PREPARATION WITH SUNSCREEN FOR SKIN PROTECTION**

| Component | Amount (% w/w) |
|---|---|
| Bis-Ethylhexyloxyphenol Methoxyphenyl Triazine | 1.00-4.00 |
| Octyldodecanol | 1.00-6.00 |
| Denat. Alcohol Type D | 1.00-15.00 |
| C₁₂-C₁₅Alkyl benzoate | 1.00-15.00 |
| Diethylamino Hydroxybenzoyl Hexyl Benzoate | 1.00-5.00 |
| Caprylic/capric Triglyceride | 1.00-60.00 |
| Ethylhexyl methoxycinnamate | 0.50-10.00 |
| Octocrylene | 0.10-5.00 |
| Simmondsia Chinensis Seed Oil | 0.10-10.00 |
| 2,4,6-Octatrienoic acid | 0.01-1.00 |

### Example 6

**DERMATOLOGICAL PREPARATION FOR THE TREATMENT OF THE FACE**

| Component | Amount (% w/w) |
|---|---|
| Propanediol | 1.00-7.00 |
| Xylitol | 0.30-1.00 |
| Cetearyl glucoside | 0.10-2.00 |
| Polyglyceryl-3 Rice Branate | 0.10-3.00 |
| Cetearyl alcohol | 0.05-1.00 |
| Disodium EDTA | 0.01-0.10 |
| 2,4,6-Octatrienoic acid potassium salt | 0.05-0.50 |
| C₁₂-C₁₅ alkyl benzoate | 1.00-5.00 |
| Ethylhexyl methoxycinnamate | 0.50-10.00 |
| Octocrylene | 0.50-5.00 |
| Butyrospermum parkii butter | 0.50-3.00 |
| Citric acid | 0.001-0.30 |
| Simmondsia Chinensis seed oil | 0.10-0.30 |
| Hydrogenated Evening Primrose Oil | 0.50-3.00 |
| Octyldodecanol | 0.50-3.00 |
| Caprylic/Capric triglyceride | 1.00-5.00 |
| Isosterayl isostearate | 0.10-5.00 |
| Beta sitosterol | 0.10-0.50 |
| Delta tocoferol | 0.05-0.20 |
| Caprylyl glycol | 0.05-0.50 |
| 1.2 Hexanediol | 0.10-0.70 |
| Sodium hydroxide | 0.001-0.20 |
| Phenoxyethanol | 0.50-0.99 |
| Perfume | 0.05-0.50 |
| Purified water | q.s. to 100.00 |

### Example 7

**DERMATOLOGICAL PREPARATION FOR SCALP SKIN**

| Component | Amount (% w/w) |
|---|---|
| Cyclopentasiloxane | 1.00-50.00 |
| Denat. Alcohol Type C | 1.00-15.00 |
| C₁₂-C₁₅ alkyl benzoate | 1.00-10.00 |
| Ethylhexyl methoxycinnamate | 1.00-5.00 |
| Octocrylene | 0.10-0.50 |
| Disiloxane | 1.00-49.00 |
| Perfume | 0.05-0.30 |
| Oleyl erucate | 0.50-3.00 |
| Dimethiconol | 0.10-10.00 |
| 2,4,6-Octatrienoic acid | 0.01-1.00 |
| Octyldodecanol | 0.01-1.00 |

### Example 8

**PROTECTIVE TREATMENT CREAM**

| Component | Amount (% w/w) |
|---|---|
| Propylene glycol | 0.50-7.00 |
| Pentylene glycol | 1.00-3.00 |
| Steareth-21 | 0.10-3.00 |
| Steareth-2 | 0.10-3.00 |
| Caprylic/capric triglyceride | 1.00-10.00 |
| Cycolopentasiloxane | 0.50-20.00 |
| Cetearyl alcohol | 0.01-2.00 |
| Octyldodecanol | 0.10-5.00 |
| Disodium EDTA | 0.01-0.10 |
| 2,4,6-Octatrienoic acidL-lysine salt | 0.05-1.00 |
| Phenoxyethanol | 0.80-1.00 |
| Sodium hydroxide | 0.001-0.20 |
| Citric acid | 0.001-0.30 |

An experimental study on cell cultures was conducted as described below, with reference to the figures in the attached drawings, to demonstrate the activity of the compounds of the present invention.

### Description of the Drawings

Figs. 1, 3, 6 show Western Blot immunofixation patterns, as described in more detail below.
Figs. 2, 4, 5, 7, 8, 9, 10, 11, 12 show diagrams relating to activity tests, as described in more detail below.

In the figures, the following meanings have to be understood:
Ctr = control
UVA or UVB = treatment with radiation only
G01 = treatment with compound of the invention only
G01/UVA = pre-treatment with compound of the invention, and subsequent UVA radiation
G01/UVB = pre-treatment with compound of the invention, and subsequent UVB radiation
UVA/G01post = UVA radiation and subsequent treatment with compound of the invention
UVB/G01post = UVB radiation and subsequent treatment with compound of the invention

### EXPERIMENTAL STUDY

The aim of the study is to evaluate the repair activity of UV induced DNA damage exercised by a compound according to said general formula (I), namely 2,4,6-trans-octatrienoic acid, identified in the description of the study herein after with the initials G01.

The study is performed on primary cultures of human keratinocytes, NHKs.

This activity has been evaluated in several respects, as follows.

### Cell viability after treatment with G01

The cells (three different cultures of keratinocytes NHKs) were plated in the specific culture medium and maintained in culture for 24 ± 2h to allow adhesion to the plate. The cells were treated with eight different concentrations of the compound of the invention G01 under evaluation, diluted in the culture medium, for 48 ± 2h. The values of cell viability for each concentration tested, resulting from the spectrophotometric reading, were compared with the value obtained from the control. The results obtained showed the absence of cellular toxicity in all eight different concentrations.

### 1) Assessment of the activity in the pre-treatment with the compound of the invention

### Cell viability after pre-treatment with G01 and subsequent UV irradiation

The cells were plated in the specific culture medium and maintained in culture for 24 ± 2h to allow adhesion to the plate. The cells were treated for 24 ± 2h with the concentrations of the compound G01 under evaluation, selected based on the previous results. Subsequently, the cells in the plates identified as +UVA and +UVB were respectively exposed, in the irradiation medium, to doses of UVA of 10 J/cm² and UVB of 25 mJ/cm², while the control plate was maintained in the dark and at room temperature for a period of time equal to the irradiation time. The irradiation medium was then replaced with the culture medium. The cell viability was measured (incorporation of NR) 24 ± 2h and 48 ± 2h after the irradiation, following examination of the morphology of the cells under a microscope, in the absence and the presence of treatment with G01 and/or UVA/UVB irradiation. The cell viability values for each condition tested were compared with the value obtained from the control and plotted on a graph to highlight any differences between the various experimental conditions.

The results obtained showed a reduction in cell viability following irradiation with UVA and UVB, and a general protective effect of cell viability, particularly 48h after irradiation, if a pre-treatment with a compound of the invention G01 was present, particularly significant at the highest concentration tested (90µM).

### Western Blot assessment of the ability to repair a DNA damage after pre-treatment with G01 and subsequent UV irradiation

The NHKs cells were plated in the specific culture medium and maintained in culture for 24 ± 2h to allow adhesion to the plate. Then, the keratinocytes were treated for 24 ± 2h with the compound of the invention G01, at concentrations of 60 µM and 90µM. Subsequently, the plates identified as +UVA, +G01/UVA, +UVB, and +G01/UVB were respectively exposed, in the irradiation medium, to doses of UVA of 10 J/cm² and UVB of 25 mJ/cm², while the control plates (Ctr) and those treated only with G01 were maintained in the dark and at room temperature for a period of time equal to the irradiation time. The irradiation medium was then replaced with the culture medium. Based on the preliminary results obtained on a broader time range, a post-treatment time of 6h was selected, after which the plates were processed for Western Blot protein determination.

For each condition tested (treatment with G01 and/or UVA/UVB irradiation), the protein expression of the phosphorylated histone γH2AX, p53 and GADD45a (growth arrest and DNA damage response gene), known markers of DNA damage induced by UV radiation, was compared with the expression in the control cells (Ctr).

The results obtained by averaging the responses of three different keratinocytes cultures have shown the protective activity of the compound G01 under examination, especially at the highest dose, as the cells pre-treated with G01 induced the protein expression of p53 and GADD45a to a lesser extent. In confirmation of these results, the pre-treated keratinocytes show, after UVB irradiation, a reduced fraction of phosphorylated histone γH2AX (fosfoγH2AX), whose expression is also associated with the amount of DNA damage.

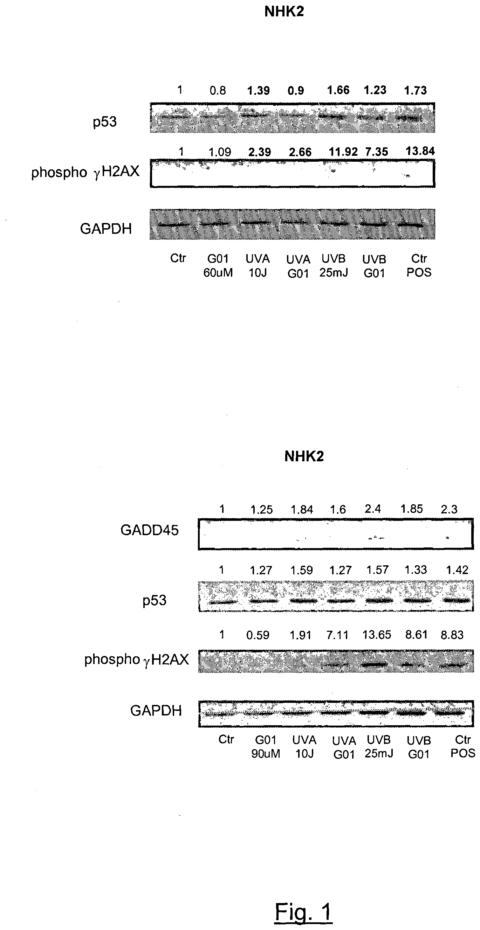

Fig.1 shows the Western Blot patterns drawn from experiments performed on one (NHK2) of the three different keratinocytes cultures described above, in which GAPDH is the constitutively expressed housekeeping, used to normalize the values of the samples.

### Flow cytometric assessment of UV induced apoptosis after a pre-treatment with G01 and subsequent UV irradiation

The NHKs cells were plated in the specific culture medium and maintained in culture for 24 ± 2h to allow adhesion to the plate. Each experimental condition was plated in duplicate. The cells were treated for 24 ± 2h with G01, in a concentration of 90µM. Subsequently, the plates identified as +UVA, +G01/UVA, +UVB, and +G01/UVB were respectively exposed, in the irradiation medium, to doses of UVA of25 J/cm² and UVB of 80 mJ/cm², while the control plates (Ctr) and those treated only with the compound under examination (G01) were maintained in the dark and at room temperature for a period of time equal to the irradiation time. The irradiation medium was then replaced with the culture medium. Based on the preliminary results obtained on a broader time range, a post-treatment time of 24h was selected, after which the plates were processed according to the procedure given in the user manual of the specific kit for annex in V flow cytometric analysis (Annex in V), a typical assay to detect the early stages of apoptosis. The positivity for annex in V in each condition tested (treatment with G01 and/or UVA/UVB irradiation) was compared with the positivity in the control cells.

The results obtained have shown the ability of G01 to reduce the apoptosis induced by with UVA and UVB radiation.

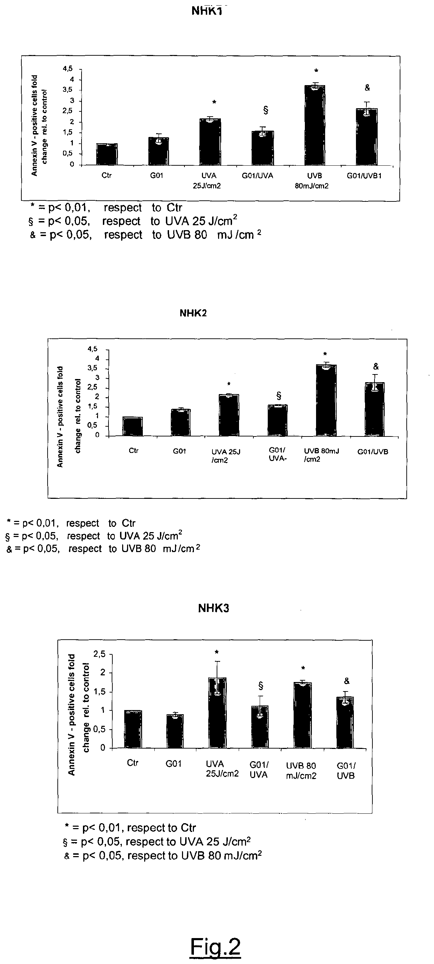

Fig. 2 shows the corresponding diagrams drawn from the experiments performed on three different keratinocytes cultures, NHK1, NHK2 and NHK3.

### Western Blot assessment of UV induced apoptosis after a pre-treatment with G01 and subsequent UV irradiation

The NHKs cells were plated in the specific culture medium and maintained in culture for 24 ± 2h to allow adhesion to the plate. Each experimental condition was plated in duplicate. The cells were treated for 24 ± 2h with the compound G01, in a concentration of 90µM. Subsequently, the cells identified as +UVA, +G01/UVA, +UVB, and +G01/UVB were respectively exposed, in the irradiation medium, to doses of UVA of 25 J/cm² and UVB of 80 mJ/cm², while the control plates and those treated only with G01 were maintained in the dark and at room temperature for a period of time equal to the irradiation time. The irradiation medium was then replaced with the culture medium. Based on the preliminary results obtained on a broader time range, a post-treatment time of 24h was selected, after which the plates were processed for Western Blot protein determination. The protein expression of non-cleaved caspase 3 in each condition tested (treatment with G01 and/or UVA/UVB irradiation) was compared with the expression in the control cells (Ctr).

The results obtained have shown the ability of G01 to reduce caspase 3 activation (corresponding to a reduction of the protein expression of the zymogen form of the protein), one of the key mediators of the apoptosis process induced by UVA and UVB.

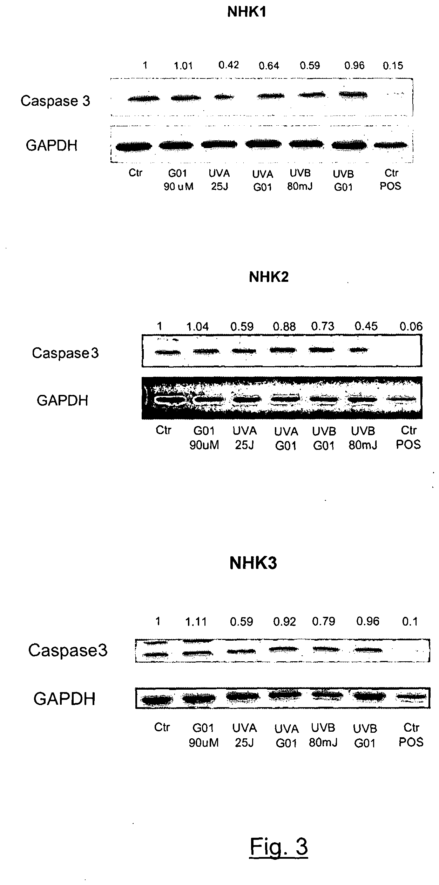

Fig. 3 shows the Western Blot patterns drawn from the experiments performed on three different keratinocytes cultures, NHK1, NHK2 and NHK3.

### ELISA assessment of DNA damage after pre-treatment with G01 and subsequent UVB irradiation: CPDs determination

The NHKs cells were plated in the specific culture medium and maintained in culture for 24 ± 2h to allow adhesion to the plate. Then, the keratinocytes were treated for 24 ± 2h with the compound G01, in a concentration of 90µM. Subsequently, the plates identified as +UVB and +G01/UVB were exposed, in the irradiation medium, to a UVB dose of 80 mJ/cm², while the control plates (Ctr) and those treated only with the compound under examination (G01) were maintained in the dark and at room temperature for a period of time equal to the irradiation time. The irradiation medium was then replaced with the culture medium. Based on the preliminary results obtained on a broader time range, post-treatment times of 6h and 24h were selected, after which the plates were processed according to the procedure described in the work instruction specific for the DNA extraction. The determination of the pyrimidine cyclobutane dimers, CPDs, was performed using a specific ELISA kit following the procedure described in the user manual. The results obtained have shown the ability of the compound G01 to promote the removal of CPDs adducts, known signal of DNA damage induced by UVB.

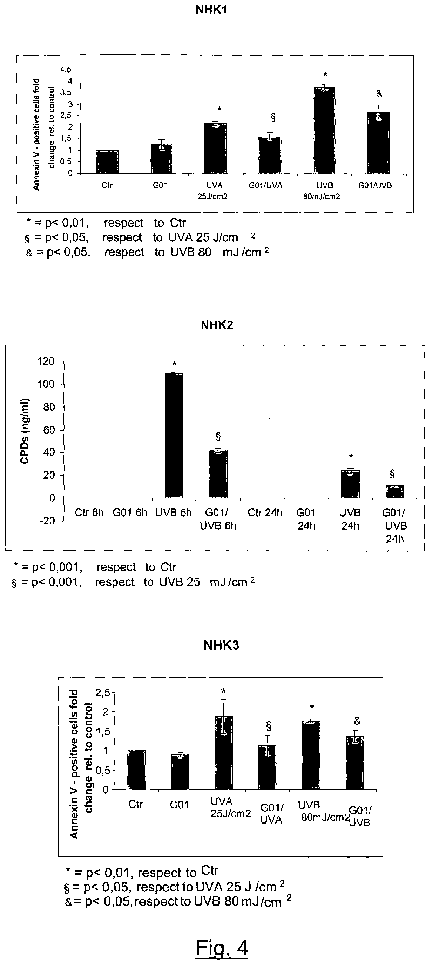

Fig. 4 shows the corresponding diagrams drawn from the experiments performed on three different keratinocytes cultures, NHK1, NHK2 and NHK3.

### 2) Assessment of the activity in the post-treatment with the compound of the invention

The following experiments were performed in order to assess the efficacy of compound G01 applied to NHKs cell cultures after irradiation with UVA and UVB.

### Cell viability after UV irradiation and subsequent treatment with G01

The cells were plated in the specific culture medium and maintained in culture for 24 ± 2h to allow adhesion to the plate. The cells in the plates identified as +UVA and +UVB were respectively exposed, in the irradiation medium, to doses of UVA of 10 J/cm² and UVB of 25 mJ/cm², while the control plate (Ctr) was maintained in the dark and at room temperature for a period of time equal to the irradiation time. The irradiation medium was then replaced with the culture medium, containing the compound G01 in concentrations selected based on the cell viability results obtained after treatment with the same compound (see above). The cell viability was measured (incorporation of NR) at a time of 24 ± 2h and 48 ± 2h after the irradiation, in the absence and the presence of UVA/UVB irradiation and/or treatment with the compound of the invention G01. The cell viability values for each condition tested were compared with the value obtained from the control and plotted on a graph to highlight any differences between the various experimental conditions.

The results obtained showed a reduction in cell viability following irradiation with UVA and UVB, and a significant protective action, particularly 48h after irradiation, of the highest concentration tested (90µM) of the compound G01.

### Flow cytometric assessment of apoptosis induced by UV irradiation and subsequent treatment with G01

The NHKs cells were plated in the specific culture medium and maintained in culture for 24 ± 2h to allow adhesion to the plate. Each experimental condition was plated in duplicate. Subsequently, the plates identified as +UVA, +G01/UVA, +UVB, and +G01/UVB were respectively exposed, in the irradiation medium, to doses of UVA of 25 J/cm² and UVB of 80 mJ/cm², while the control plates (Ctr) and those treated only with the compound under examination (G01) were maintained in the dark and at room temperature for a period of time equal to the irradiation time. The irradiation medium was then replaced with the culture medium containing the compound G01 in a concentration of 90µM. Based on the preliminary results obtained on a broader time range, a post-treatment time of 24h was selected, after which the plates were processed according to the procedure given in the user manual of the specific kit for annex in V flow cytometric analysis. The positivity for annex in V in each condition tested (treatment with the compound of the invention under examination and/or UVA/UVB irradiation) was compared with the positivity in the control cells.

The results obtained have shown the ability of the compound G01 to reduce the apoptosis induced by irradiation with UVA and UVB.

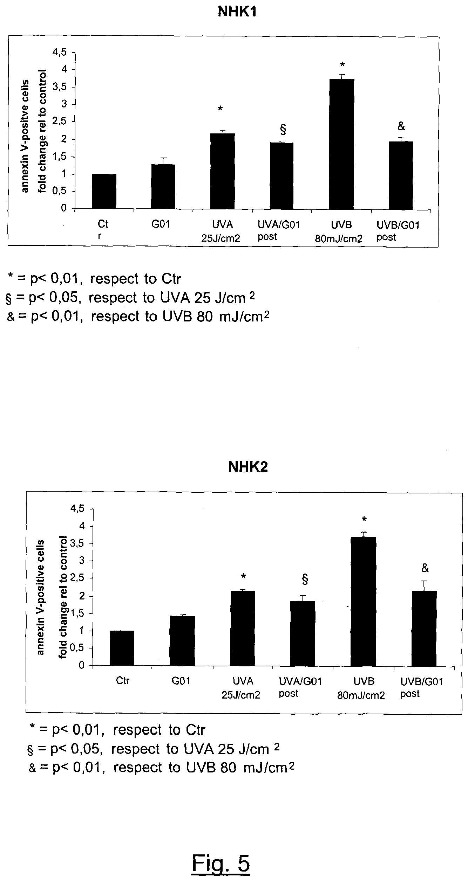

Fig. 5 shows the corresponding diagrams drawn from the experiments performed on two different keratinocytes cultures, NHK1 and NHK2.

### Western Blot assessment of apoptosis induced by UV irradiation and subsequent treatment with G01

The NHKs cells were plated in the specific culture medium and maintained in culture for 24 ± 2h to allow adhesion to the plate. Subsequently, the plates identified as +UVA, +G01/UVA, +UVB, and +G01/UVB were respectively exposed, in the irradiation medium, to doses of UVA of 25 J/cm² and UVB of 80 mJ/cm², while the control plates (Ctr) and those treated only with the compound under examination (G01) were maintained in the dark and at room temperature for a period of time equal to the irradiation time. The irradiation medium was then replaced with the culture medium containing the compound G01, in a concentration of 90µM. Based on the preliminary results obtained on a broader time range, a post-treatment time of 24h was selected, after which the plates were processed for Western Blot protein determination. The protein expression of non-cleaved caspase 3 in each condition tested (treatment with the compound G01 under examination and/or UVA/UVB irradiation) was compared with the expression in the control cells.

The results obtained have shown the ability of the compound of the invention to reduce caspase 3 activation (corresponding to a reduction of the protein expression of the zymogen form of the protein), one of the key mediators of the apoptosis process induced by UVA and UVB.

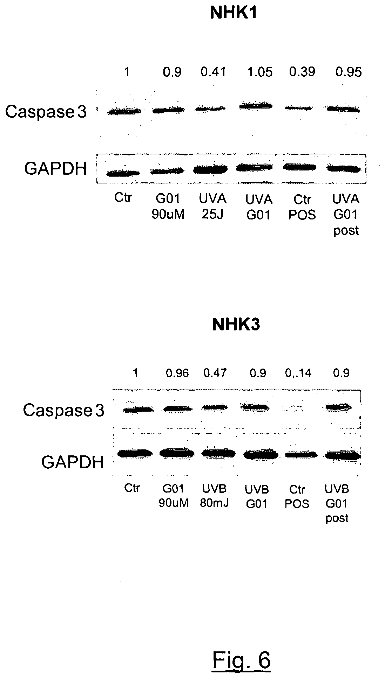

Fig. 6 shows the Western Blot patterns drawn from the experiments performed on two different keratinocytes cultures, NHK1 and NHK3.

### ELISA assessment of the damage induced by UVB irradiation and subsequent treatment with G01: CPDs determination

The NHKs cells were plated in the specific culture medium and maintained in culture for 24 ± 2h to allow adhesion to the plate. Then, the plates identified as +UVB and +G01/UVB were respectively exposed, in the irradiation medium, to a UVB dose of 25 mJ/cm², while the control plates (Ctr) and those treated only with the compound under examination (G01) were maintained in the dark and at room temperature for a period of time equal to the irradiation time. The irradiation medium was then replaced with the culture medium containing the compound G01, in a concentration of 90µM. Based on the preliminary results obtained on a broader time range, post-treatment times of 6h and 24h were selected, after which the plates were processed according to the procedure described in the work instruction specific for the DNA extraction. The CPDs determination was performed using a specific ELISA kit following the procedure described in the user manual.

The results obtained have shown the ability of the compound G01 under examination to promote the removal of CPDs products, known signal of DNA damage induced by UVB.

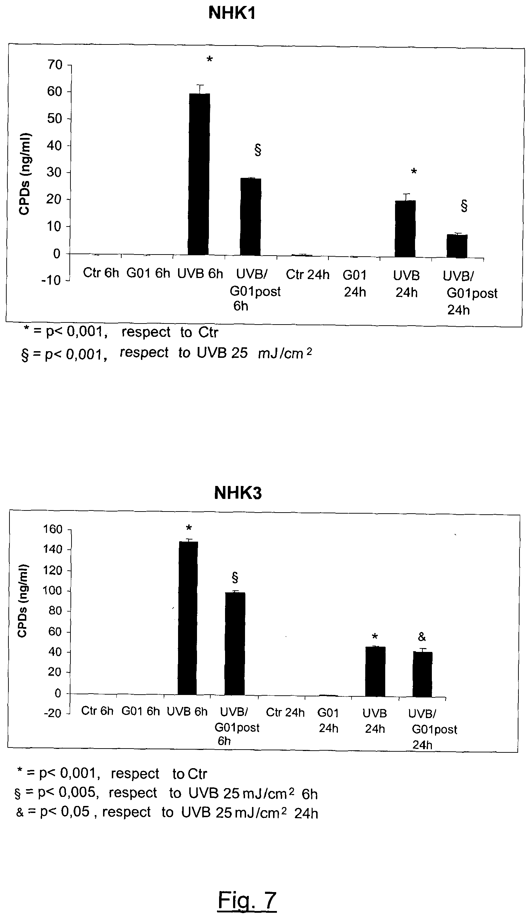

Fig. 7 shows the corresponding diagrams drawn from the experiments performed on two different keratinocytes cultures, NHK1 and NHK3.

### ELISA assessment of the damage induced by UVA irradiation and subsequent treatment with G01: 8-OHdG determination

The NHKs cells were plated in the specific culture medium and maintained in culture for 24 ± 2h to allow adhesion to the plate. Then, the plates identified as +UVA and +UVA/G01 postwere respectively exposed, in the irradiation medium, to a UVA dose of 10 J/cm². The irradiation medium was then replaced with the culture medium containing the compound of the invention G01, in a concentration of 90µM. Based on preliminary results obtained on a broader time range, post-treatment times of 6h and 24h were selected, after which the plates were processed according to the procedure described in the work instruction specific for the DNA extraction. The determination of 8-OHdG, marker of DNA oxidative damage induced by UVA, was performed using a specific ELISA kit following the procedure described in the user manual.

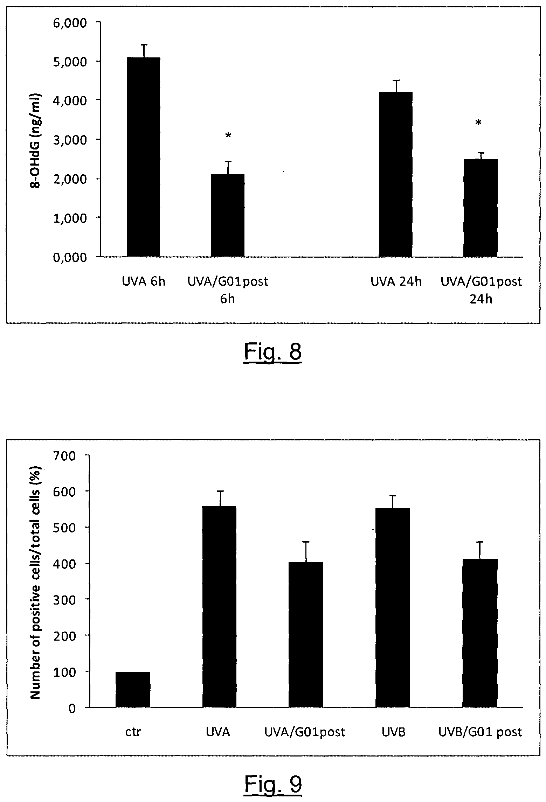

The results obtained are summarized in the graph of Fig. 8. They show, in the cells irradiated and post-treated with the compound of the invention, a significant reduction in the amount of 8-OHdG produced by UVA radiation at both post-treatment times compared to the cells only irradiated and not treated with the compound.

### Immunofluorescence assessment of the ability to repair a DNA damage following UVA or UVB irradiation and subsequent treatment with G01

The NHKs cells were plated in the specific culture medium and maintained in culture for 24 ± 2h to allow adhesion to the plate. Subsequently, the plates identified as +UVA, +UVA/G01 post, UVB, and +UVB/G01 post were respectively exposed, in irradiation medium, to doses of UVA of 10 J/cm² or UVB of 25 mJ/cm². The irradiation medium was then replaced with the culture medium containing the compound of the invention G01, in a concentration of 90µM. Based on preliminary results obtained on a broader time range, a post-treatment time of 24h was selected, after which the plates were processed for the immunofluorescence determination of the protein expression of the phosphorylated histone γH2AX, whose expression is associated to the amount of DNA damage (double strand breaks). The protein expression of the phosphorylated histone γH2AX after treatment with the compound of the invention G01, or only after irradiation with UVA/UVB, was compared with the expression in the control cells.

The results obtained are summarized in the graph of Fig. 9. They show, in the cells irradiated and post-treated with the compound of the invention, a significant reduction in the fraction of phosphorylated histone γH2AX compared to the cells only irradiated and not treated with the compound.

### Western Blot assessment of the ability to repair a DNA damage following UVB irradiation and subsequent treatment with G01

The NHKs cells were plated in the specific culture medium and maintained in culture for 24 ± 2h to allow adhesion to the plate. Subsequently, the cells identified as +UVB and +G01/UVB post were exposed, in the irradiation medium, to a UVB dose of 25 mJ/cm². The irradiation medium was then replaced with the culture medium containing the compound of the invention G01, in a concentration of 90µM. Based on preliminary results obtained on a broader time range, a post-treatment time of 24h was selected, after which the plates were processed for Western Blot protein determination. The protein expression of p53, phospho-p53, GADD45a, and phosphorylated histone γH2AX was measured in the condition of UVB irradiation.

### Western Blot analysis at pre-set post-treatment times "time course" of the expression levels of p53, phospho-p53 and GADD45a after UVB irradiation and subsequent treatment with G01

The NHKs cells were plated in the specific culture medium and maintained in culture for 24 ± 2h to allow adhesion to the plate. Subsequently, the plates identified as +UVB and +G01/UVB post were exposed, in the irradiation medium, to a UVB dose of 25 mJ/cm². The irradiation medium was then replaced with the culture medium containing the compound of the invention G01, in a concentration of 90µM. Post-treatment times of 1-3-6-24h were selected, after which the plates were processed for Western Blot protein determination. The protein expression of p53, phospho-p53, and GADD45a after UVB irradiation and post-treatment with the compound of the invention G01 was compared with the expression in the control cells (Ctr).

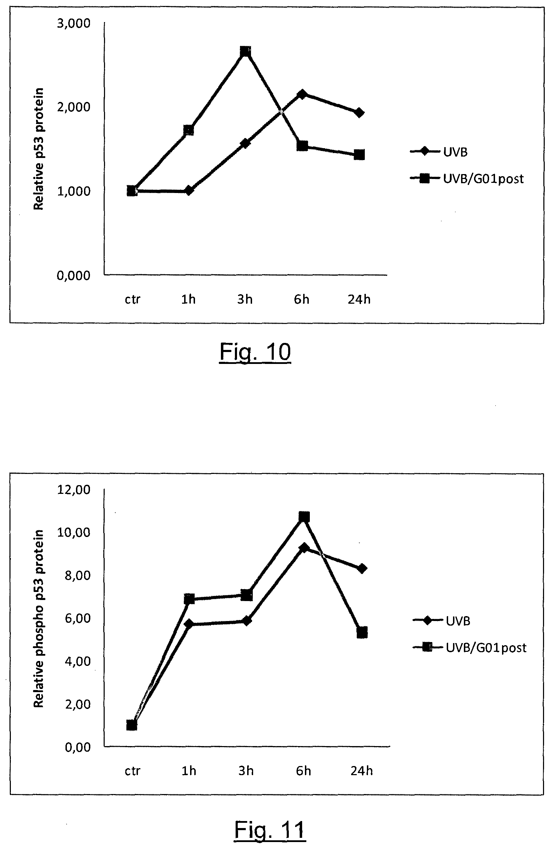

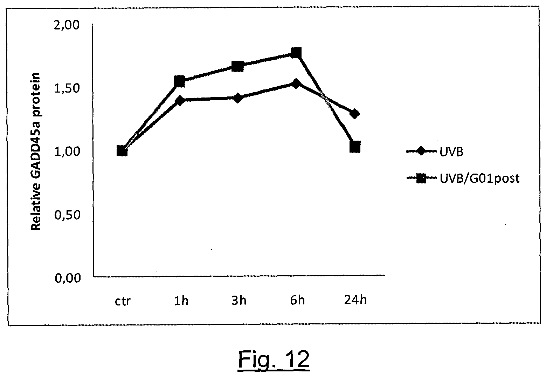

The results obtained are summarized in the diagrams of Figures 10 to 12, which represent the densitometries of the Western Blot electrophoretic patterns of immunofixation obtained at the above-specified times. They show that the post-treatment with the compound of the invention of cells exposed to UVB causes an acceleration of the increase in the levels of p53, phospho-p53. and GADD45a in the shorter times, resulting in a rapid activation of the DNA damage repair mechanisms. As final general result, this action causes a decrease in the expression of the markers of DNA damage at 24h after UVB irradiation and, thus, a cytoprotective effect.

Overall, the experimental results obtained in the post-treatment of UV irradiation show, for the compound of the invention, an effective activity against DNA damage, similar to that demonstrated above in the pre-treatment before UV irradiation, particularly for what concerns key parameters such as preservation of cell viability, positivity to annex in V, inhibition of caspase 3 protein expression, CPDs and 8-OHdG inhibition, markers of DNA damage.

Moreover, in cells exposed to UVB irradiation, the post-treatment with compounds of the invention results in a timely activation of the DNA damage repair mechanisms. This results in a decrease of the expression of the markers of damage at 24h after the irradiation, and a cytoprotective effect.

Taken together, the experimental data therefore envisage an anticancer use of the compounds of general formula (I), both in the prevention of a cancer that may develop and in the treatment of an already developed cancer, as a result of a human DNA damaged induced by UV radiation.

As secondary use, dependent on the anticancer one, particularly in the case of prevention of DNA damage caused by UV radiation, a use as active ingredient is envisaged in compositions directed to the photoprotection of the skin, including the scalp skin, from UVA and UVB, to prevent DNA damage potentially developable in a cancer.

For this reason, a further object of the present invention is also such photoprotective use dependent on the one described above.

## Claims

1. Compounds of general formula (I):
CH₃-(CH=CH)ₙ-R (I)
wherein n = 3, 5, 7; R is selected from -CO-OR', -CO-O⁽⁻⁾, R' being selected from H, C₁-C₂₂ alkyl or alkenyl, aryl or aralkyl, or sugars; and pharmaceutically acceptable salts thereof, preferably such as sodium, potassium, lysine salts,
each compound of general formula (I) being used as such or in admixture with one or more of the other ones,
for use in the prevention or treatment of skin cancer resulting from DNA damage produced by UV radiation in the human keratinocytes, wherein the compounds topically administered to the skin produce at least the following combined effects: preservation of cell viability, positivity to annex in V, apoptosis inhibition, inhibition of caspase-3 protein expression, inhibition of CPDs and 8-OHdG.

2. Compounds for use according to claim 1, intended for the further use of obtaining, together, skin photoprotection, including the scalp, from DNA damage produced by UV radiation.

3. A composition for use according to claims 1 and 2, wherein the active ingredient is a compound of general formula (I) used as such or in admixture with one or more of the other compounds.

4. A composition for use according to claim 3, wherein said active ingredient is 2,4,6-octatrienoic acid.

5. A composition for use according to claim 3, wherein said active ingredient is a 2,4,6-octatrienoic acid salt.

6. A composition for use according to claim 3, wherein said active ingredient is 2,4,6-octatrienoic acid sodium salt.

7. A composition for use according to claim 3, wherein said active ingredient is 2,4,6-octatrienoic acid potassium salt.

8. A composition for use according to claim 3, wherein said active ingredient is 2,4,6-octatrienoic acid L-lysine salt.

9. A composition for use according to claim 3, wherein said active ingredient is 2,4,6-octatrienoic acid ethyl ester.

10. A composition for use according to claim 3, wherein said active ingredient is 2,4,6-octatrienyl ester of 2,4,6-octatrienoic acid.

11. A composition for use according to claim 3, wherein said active ingredient is 2,4,6-octatrien-1-ol acetate.

12. A composition for use according to claim 3, wherein said active ingredient is 2,4,6-octatrien-1-ol benzoate.

13. A composition for use according to claim 3, wherein said active ingredient is a mixture of two or more of the compounds of formula (I).

14. A composition for use according to any one of claims 3 to 13, comprising said active ingredient in an amount from 10⁻⁶ to 3x10⁻² mol/100g.

15. A composition for use according to claims 3 to 5, comprising said active ingredient in an amount of 90µM.

16. A composition for use according to any one of claims 3 to 15, comprising said active ingredient formulated with any suitable excipient for topical administration on the human skin or scalp.

## Patentansprüche

1. Zusammensetzungen der allgemeinen Formel (I):
CH₃-(CH=CH)ₙ-R (I)
wobei n = 3, 5, 7; R ausgewählt wird aus -CO-OR', -CO-O⁽⁻⁾, R' ausgewählt wird aus H, C₁-C₂₂ Alkyl oder Alkenyl, Aryl oder Aralkyl, oder Zuckern; und pharmazeutisch akzeptablen Salzen davon, vorzugsweise Natrium, Kalium, Lysinsalze,
wobei jede Zusammensetzungen der allgemeinen Formel (I) als solche oder als Mischung mit einer oder mehreren der anderen verwendet wird,
bei der Verwendung zur Prävention oder Behandlung von Hautkrebs, der aus DNA-Schädigung der menschlichen Keratinozyten durch UV-Strahlung resultiert,
wobei die topisch auf die Haut applizierten Zusammensetzungen zumindest die folgenden kombinierten Effekte bewirken: Erhaltung der Zellviabilität, Positivität bzgl. dem Anhang in V, Apoptose-Inhibition, Inhibition von Caspase-3-Proteinexpression, Inhibition von CPDs und 8-OHdG.

2. Zusammensetzungen zur Verwendung gemäß Anspruch 1, vorgesehen zur weiteren Verwendung zum gemeinsamen Erhalt von Haut-Photoprotektion, inklusive der Kopfhaut, und DNA-Schädigung durch UV-Strahlung.

3. Zusammensetzung zur Verwendung gemäß Anspruch 1 und 2, wobei der Wirkstoff eine Zusammensetzung der allgemeinen Formel (I) ist, die als solche oder als Mischung mit einer oder mehreren der anderen Zusammensetzungen verwendet wird.

4. Zusammensetzung zur Verwendung gemäß Anspruch 3, wobei der Wirkstoff 2,4,6-Oktatriensäure ist.

5. Zusammensetzung zur Verwendung gemäß Anspruch 3, wobei der Wirkstoff ein 2,4,6-Oktatriensäuresalz ist.

6. Zusammensetzung zur Verwendung gemäß Anspruch 3, wobei der Wirkstoff 2,4,6-Oktatriensäurenatriumsalz ist.

7. Zusammensetzung zur Verwendung gemäß Anspruch 3, wobei der Wirkstoff 2,4,6-Oktatriensäurekaliumsalz ist.

8. Zusammensetzung zur Verwendung gemäß Anspruch 3, wobei der Wirkstoff 2,4,6-Oktatriensäure-L-Lysinsalz ist.

9. Zusammensetzung zur Verwendung gemäß Anspruch 3, wobei der Wirkstoff 2,4,6-Oktatriensäure-Ethylester ist.

10. Zusammensetzung zur Verwendung gemäß Anspruch 3, wobei der Wirkstoff 2,4,6-Oktatrienylester der 2,4,6-Oktatriensäure ist.

11. Zusammensetzung zur Verwendung gemäß Anspruch 3, wobei der Wirkstoff 2,4,6-Oktatrien-1-ol-Azetat ist.

12. Zusammensetzung zur Verwendung gemäß Anspruch 3, wobei der Wirkstoff 2,4,6-Oktatrien-1-ol-Benzoat ist.

13. Zusammensetzung zur Verwendung gemäß Anspruch 3, wobei der Wirkstoff eine Mischung von zwei oder mehreren Zusammensetzungen der Formel (I) ist.

14. Zusammensetzung zur Verwendung gemäß einem der Ansprüche 3 bis 13, die den Wirkstoff in einer Menge von 10⁻⁶ bis 3x10⁻² mol / 100g aufweist.

15. Zusammensetzung zur Verwendung gemäß den Ansprüchen 3 bis 5, die den Wirkstoff in einer Menge von 90 µM aufweist.

16. Zusammensetzung zur Verwendung gemäß einem der Ansprüche 3 bis 15, zur topischen Applikation auf der menschlichen Haut oder Kopfhaut, die den Wirkstoff, formuliert mit einem geeigneten Hilfsstoff, aufweist.

## Revendications

1. Composés de formule générale (I) :
CH₃-(CH=CH)ₙ-R (I)
dans lesquels n = 3, 5, 7 ; R est choisi parmi -CO-OR', -CO-O⁽⁻⁾, R' étant choisi parmi H, un alkyle ou un alcényle en C₁ à C₂₂, un aryle ou un aralkyle, ou des sucres ; et des sels pharmaceutiquement acceptables de ceux-ci, de préférence tels que des sels de sodium, de potassium, de lysine,
chaque composé de formule générale (I) étant utilisé tel quel ou en mélange avec un ou plusieurs des autres,
pour utilisation dans la prévention ou le traitement du cancer de la peau résultant des dommages de l'ADN produits par un rayonnement UV dans les kératinocytes humains, dans lesquels les composés administrés topiquement sur la peau produisent au moins les effets combinés suivants : préservation de la viabilité cellulaire, positivité à l'annexine V, inhibition de l'apoptose, inhibition de l'expression de la protéine caspase-3, inhibition des CPDs et de la 8-OHdG.

2. Composés pour utilisation selon la revendication 1, conçus pour l'utilisation supplémentaire consistant à obtenir, conjointement, une photoprotection de la peau, incluant le cuir chevelu, contre les dommages de l'ADN produits par un rayonnement UV.

3. Composition pour utilisation selon les revendications 1 et 2, dans laquelle le principe actif est un composé de formule générale (I) utilisé tel quel ou en mélange avec un ou plusieurs des autres composés.

4. Composition pour utilisation selon la revendication 3, dans laquelle ledit principe actif est l'acide 2,4,6-octatriénoïque.

5. Composition pour utilisation selon la revendication 3, dans laquelle ledit principe actif est un sel de l'acide 2,4,6-octatriénoïque.

6. Composition pour utilisation selon la revendication 3, dans laquelle ledit principe actif est un sel sodique de l'acide 2,4,6-octatriénoïque.

7. Composition pour utilisation selon la revendication 3, dans laquelle ledit principe actif est un sel potassique de l'acide 2,4,6-octatriénoïque.

8. Composition pour utilisation selon la revendication 3, dans laquelle ledit principe actif est un sel de L-lysine de l'acide 2,4,6-octatriénoïque.

9. Composition pour utilisation selon la revendication 3, dans laquelle ledit principe actif est un ester éthylique de l'acide 2,4,6-octatriénoïque.

10. Composition pour utilisation selon la revendication 3, dans laquelle ledit principe actif est l'ester 2,4,6-octatriénylique de l'acide 2,4,6-octatriénoïque.

11. Composition pour utilisation selon la revendication 3, dans laquelle ledit principe actif est l'acétate de 2,4,6-octatrién-1-ol.

12. Composition pour utilisation selon la revendication 3, dans laquelle ledit principe actif est le benzoate de 2,4,6-octatrién-1-ol.

13. Composition pour utilisation selon la revendication 3, dans laquelle ledit principe actif est un mélange de deux des composés de formule (I) ou plus.

14. Composition pour utilisation selon l'une quelconque des revendications 3 à 13, comprenant ledit principe actif en une quantité de 10⁻⁶ à 3 x 10^{- 2} mol/100 g.

15. Composition pour utilisation selon les revendications 3 à 5, comprenant ledit principe actif en une quantité de 90 µM.

16. Composition pour utilisation selon l'une quelconque des revendications 3 à 15, comprenant ledit principe actif formulé avec tout excipient approprié pour une administration topique sur la peau ou le cuir chevelu humains.
